# EUROPEAN PATENT APPLICATION

(11) **EP 3 407 062 A1**
(43) Date of publication of application: **28.11.2018**
(21) Application number: 17172215.0
(22) Date of filing: 22.05.2017
(51) Int. Cl.: G01N 33/00, G01N 21/77

(54) **HYDROGEN SENSING SYSTEM WITH DIELECTRIC WAVEGUIDE**

(71) Applicant: ABB Schweiz AG, 5400 Baden (CH)
(72) Inventor: GREMAUD, Robin, 8048 Zürich (CH); VAN MECHELEN, Jacobus Lodevicus Martinus, 8104 Regensdorf (CH); MÜLLER, Georg, 8152 Glattpark (CH)
(74) Representative: ABB Patent Attorneys

(57) **Abstract**

A hydrogen sensor for detecting hydrogen in a medium to be monitored is provided. The sensor comprises a hybrid photonic-plasmonic waveguide (HPWG), comprising a dielectric waveguide, having a substantially rectangular cross-section; a buffer layer, stacked on top of the dielectric waveguide; a sensing layer stacked on the buffer layer, being metallic or semi-metallic and having a refractive index and/or absorption coefficient which is dependent on a hydrogen partial pressure at the sensing layer; and optionally a catalytic layer stacked on the sensing layer. The hydrogen sensor is configured such that a medium to be monitored is in physical contact to the sensor, and configured such that hydrogen in the medium may permeate into the sensing layer, and wherein at least one mode of a propagating wave in the HPWG is affected by a change in the refractive index and/or absorption coefficient of the sensing layer due to a change in the hydrogen concentration in the medium to be monitored. Further, a detection/sensing system employing such sensors, and respective methods are provided.

## Description

### Field

Aspects of the present disclosure relate to a hydrogen sensor, a hydrogen sensing system employing the same, and to an electrical device including such a sensing system, which is also suitable to detect gases other than hydrogen. In particular, aspects relate to a hydrogen sensor and a hydrogen sensing/detection system employing hybrid photonic-plasmonic waveguides, which is/are suitable for detecting hydrogen in liquid-filled electrical equipment.

### Technical background

Insulation-liquid-filled electrical equipment, such as oil-filled shunt reactors, bushings, and especially transformers such as power and distribution transformers, are filled with insulation liquid, in particular oil, for cooling and electrical insulation purposes. Faults inside the electrical equipment as well as degradation of the insulation liquid and of other insulation components such as insulation paper provided within the electrical equipment can form decomposition gasses which mainly dissolve into the liquid. This is valid for equipment employing both mineral oil and oil from natural sources.

It is important to detect such faults, errors and degradations early, since especially transformers are important components of the electrical grid, and their failure can be very costly. Hence, a transformer is supposed to operate continuously and as error-free as possible over many years or even decades.

The quantity and composition of the decomposition gases is dependent on the underlying defect: A large fault with high energy content, such as rapid overheating or arcing, causes large amounts of gas produced in a short period of time, whereas the amount of gas produced by a small fault may be relatively smaller. Also, the relative concentrations of the different gasses dissolved might indicate the specific type of fault. Thus, if the nature and amount of individual gases dissolved in the insulation liquid are known, the occurrence of a change of the concentration of a specific gas in the oil can be used to identify an electrical fault in the equipment. It is known that one of the most important indicators for electrical failure in oil insulated transformers is the occurrence of hydrogen gas dissolved in the oil, which is for example produced at a faulty portion of an insulation of a winding of the transformer, by thermal or electrical decomposition of the oil. For this reason, it is desirable that such errors, which may eventually cause complete failure of the transformer, can be detected as early as possible by identifying a rise in hydrogen concentration. This should ideally be possible at a stage when appropriate counter-measures may still be taken before serious and potentially costly malfunction occurs.

At a very early stage of such an electrical fault, only a very small amount of hydrogen gas may be produced, which dissolves in the oil and thus a concentration of dissolved hydrogen builds up in the oil over a longer period of time - whereby the hydrogen concentration in the oil may, at least during an early phase of the failure, even be below a threshold at which it can be detected with most known detection methods.

For detecting even small traces of hydrogen in transformer oil, since a few years on-line hydrogen monitoring devices are known which include thin-film based fiber-optic sensors, wherein a sensing material changes its optical properties upon an exposure to hydrogen dissolved in the oil. One such system for detecting hydrogen gas is described as an optical switching device in WO 2007 049965 A1. One of the sensing materials frequently used in hydrogen sensors is Palladium (Pd), which is reacting at too high a hydrogen partial pressure for application in an electrical transformer - where typically, a first warning level of about 100 ppm hydrogen in oil is desirable according to literature. Monitoring below this level can also be desirable, as healthy transformers exhibit hydrogen levels between about 50 ppm and 150 ppm, according to IEC 60599.

Other, more appropriate materials for the application in hydrogen sensors for transformers are, for example, disclosed in EP 2778660 A1 as a Mg-transition metal, in particular Mg-Ti. WO 2016026803 A1 describes sensing layers comprising Mg-Ni-Zr. In most cases, the change of the optical reflectance or absorption of a thin film metal layer, depending on the varying hydrogen partial pressure in the transformer oil, is determined.

The known solutions leave room for improvement. In view of the above and for other reasons, there is a need for the present invention.

### Summary of the invention

In view of the above, a hydrogen sensor for detecting hydrogen in a medium to be monitored according to claim 1, and a method of detecting hydrogen in a medium to be monitored according to claim 14 are provided.

According to a first aspect, a hydrogen sensor for detecting hydrogen in a medium to be monitored is provided. The sensor comprises a hybrid photonic-plasmonic waveguide (HPWG), comprising a dielectric waveguide having a substantially rectangular cross-section; a buffer layer, stacked on top of the dielectric waveguide; a sensing layer stacked on the buffer layer, being metallic or semi-metallic and having a refractive index and/or absorption coefficient which is dependent on a hydrogen partial pressure at the sensing layer. The hydrogen sensor is configured such that a medium to be monitored is in physical contact to the sensor, and configured such that hydrogen in the medium may permeate into the sensing layer, and wherein at least one mode of a propagating wave in the HPWG is affected by a change in the refractive index and/or absorption coefficient of the sensing layer due to a change in the hydrogen concentration in the medium to be monitored.

According to a second aspect, a method of detecting hydrogen in a medium to be monitored is provided. It comprises providing a hydrogen sensor according to the first aspect, which comprises a hybrid photonic-plasmonic waveguide (HPWG) with a sensing layer, wherein the hydrogen sensor is configured such that a medium to be monitored is in physical contact with the sensor; and coupling an electromagnetic wave into the HPWG. The hydrogen sensor is configured such that a medium to be monitored is in physical contact to the sensor, wherein hydrogen permeates into the sensing layer and influences at least one mode of a propagating wave in the HPWG via the refractive index and/or absorption coefficient of the sensing layer.

A catalytic layer may optionally be stacked on the sensing layer, or the catalytic property is achieved via the choice of material of the sensing layer, which is per se known in the art. Further, generally different types of barrier / protection layers may be stacked on the sensing layer or catalytic layer in order to protect the sensor from sensor-poisonous or damaging components in the medium to be monitored, e.g., transformer oil.

Generally, a HPWG structure according to aspects comprises a dielectric waveguide, a buffer layer and a metallic or semi-metallic layer. The HPWG acts directly as a chemical sensor, wherein the signal is independent of intensity variations of the incoming light. Thus, the sensor is optically self-calibrated. The (semi-)metallic part is designed as a sensing layer stack that changes its effective light absorption coefficient and/or its refractive index upon chemical changes of the environment. The HPWG is generally, according to embodiments, used as polarization selector or as a ring resonator. In particular, optically self-calibrated HPWG structures may be used as sensors for hydrogen in transformer oil.

Further advantages, features, aspects and details that can be combined with embodiments described herein are evident from the dependent claims, the description and the drawings.

The provided optical sensor and detection system employing it, as well as the described methods for operation and uses thereof in electrical devices, all according to embodiments of the invention, allow for, amongst other advantages, an optically self-calibrated operation.

While in this disclosure, most embodiments and examples are directed to a hydrogen sensor and hydrogen sensing system and method, employed for detecting hydrogen in a fluid being transformer oil, these are only exemplary, non-limiting examples. In embodiments, the concept may also be employed for the detection of other gases than hydrogen, and in other fluids than transformer oil. As an example, concentrations of a gas other than hydrogen in a gas mixture - or a liquid - may be detected. In such embodiments, the material of the sensing layer may be configured according to the gas to be detected, which is a standard task for the skilled person.

### Brief description of the Figures

More details will be described in the following with reference to the figures, wherein
- Fig. 1: is a schematic view on the simulated field lines of the TE1 mode (left) and the TM1 mode (right) in a dielectric waveguide provided on a substrate;
- Fig. 2: is a schematic view on the simulated electric field strength of the TE1 mode (left) and the TM1 mode in a waveguide provided on a substrate, with white colour indicating a high field, black colour indicating a low field;
- Fig. 3: is a schematic front view of a sensor according to embodiments disclosed herein;
- Fig. 4A: is a schematic side view of the sensor of Fig. 3 as part of a hydrogen sensing system according to embodiments;
- Fig. 4B: shows four sensor configurations according to embodiments;
- Fig. 5: shows various working ranges of sensors according to embodiments;
- Fig. 6: schematically shows exemplary simulations of the electric field strengths in an exemplary sensor such as shown in Fig. 3 and Fig. 4A, with white colour indicating a high field, black colour indicating a low field;
- Fig. 7: shows simulated field distributions for the TE1 mode and TM1 mode in a dielectric waveguide of sensors according to embodiments sucvh as shown in Fig. 3 and Fig. 4A;
- Fig. 8: shows the change in transmission in dependency from a hydrogen partial pressure for sensors according to embodiments, in comparison to an exemplary Pd-based sensor;
- Fig. 9: shows a sensor according to further embodiments and as part of a sensing system according to embodiments;
- Fig. 10: shows a shift of the resonance frequency in a sensor such as shown in Fig. 9 as a function of the difference in refractive index between a sensor and dielectric substrate;
- Fig. 11: shows a more detailed view on a sensor similar to the one shown in Fig. 9;
- Fig. 12: shows a change in optical transmission in dependency from a shift of the wavelength coupled into the sensor of Fig. 11.

### Detailed Description of Aspects of the Invention

As used herein, the term "fluid" is intended to be both representative for gases and liquids. It is, however, mainly used to be representative of an insulation liquid, particularly an oil, which is part of the insulation and/or cooling system of an electrical device, more particularly of a power transformer.

As used herein, the term "calibration" is intended to mean that in a hydrogen sensing system according to embodiments, apparative and/or process-inherent measures are taken to minimize or ideally nullify an influence of - per se typically unknown - intensity fluctuations of the light source(s), as well as of light intensity fluctuations due to variations in the optical couplings or the like, from having an influence on the accuracy on the measurement results of the hydrogen sensing system. A continuous, or frequently carried out, calibration or self-calibration can be seen as advantageous, as this takes into account intensity variations or fluctuations in real time or at least nearly in real time, thus enhancing precision and reliability of the readings produced by the system.

As used herein, the expression "the ratio" in conjunction with the measured intensities of two polarization components of light means either the mathematical ratio between the intensities of the two components, or can as well mean to calculate a difference of the two intensities, divided by the sum of the two intensities, which by definition also yields "the ratio". In embodiments described herein, both variants/meanings are equally applicable. It goes without saying that the skilled person may find other ways of calculating a "ratio", in the broadest possible sense, from and of the two measured intensities, without departing from the technical meaning and application of the process as described herein. Hence, such variations are regarded to also fall into the scope of the present disclosure.

As used herein, metal alloys defined by a formula with atomic percentage values typically adding to 100 percent, such as, for example, Mg₅₂Ni₂₀Zr₃₅, are meant to also include substances with a composition deviating from that with the exact numbers provided. Typically, alloys having a composition wherein each number, independently from each other, has a tolerance of +/-15 percent, are still regarded to fall under the metal alloy provided by provision of the exact formula, such as the example above, also if the single numbers do not add up to 100 in total. Also, as used herein, such alloys may comprise further, non-named substances such as chemical elements of smaller amounts, such as up to about 2 percent each, but not more than about 10 percent in total.

In the following, some aspects of the invention are described in detail. Aspects and parts of aspects are independent of each other and can be combined in any manner. For example, any aspect or embodiment described in this document can be combined with any other aspect or embodiment, as long as the combinations achieved are technically feasible, or unless the contrary is mentioned.

First, some general possible aspects relating to the hydrogen sensor are described. The sensor is suitable and adapted for sensing a status condition of an insulation-liquid-filled electrical equipment. Herein, electrical equipment refers to any equipment such as shunt reactors, bushings and transformers. The invention is particularly suited for the insulation liquid being insulation oil, be it on a mineral basis, or from organic sources, such as e.g., palm oil. The invention is further particularly suited for the electrical equipment being a transformer, such as a power or distribution transformer, and even more particularly for an oil-filled/oil-insulated power transformer.

The status condition of the electrical equipment is herein expressed by the hydrogen content (or hydrogen concentration) of the insulation liquid, which is a reliable indicator of various conditions, in particular fault conditions. The hydrogen content is defined as the amount of hydrogen dissolved in the insulation liquid (e.g., in ppm). A hydrogen sensitive layer (henceforth also called sensing layer) of an optical sensor is arranged in communication with the fluid (insulation liquid, oil), and is preferably immersed in the insulation liquid, so that the amount of hydrogen dissolved in the insulation liquid results in a characteristic partial pressure of hydrogen at the optical sensor, this partial pressure being a function of the hydrogen content (in ppm) in the insulation liquid. This relation may depend on additional parameters such as the temperature of the insulation liquid and/or of the hydrogen sensitive layer, and on the type of oil used in the transformer. Herein, the term "hydrogen" may refer to hydrogen molecules or atoms (which may be radicals). As used herein, the sensing layer "being in communication with a fluid" means that the gaseous components of interest present in the fluid, in particular hydrogen, may reach the sensing layer, even if other layers for catalysis, protection, or the like, are located/stacked between the sensing layer and the fluid. The metal alloy of the sensing layer reacts with the hydrogen from the fluid which diffuses through a protection layer (if present), and thereby builds a metal-alloy hydride system. The latter reaction is a reason for the change in dielectric/optical properties of the sensing layer when hydrogen is present, which is used for a hydrogen detection, or if desired the detection of other gases, in embodiments.

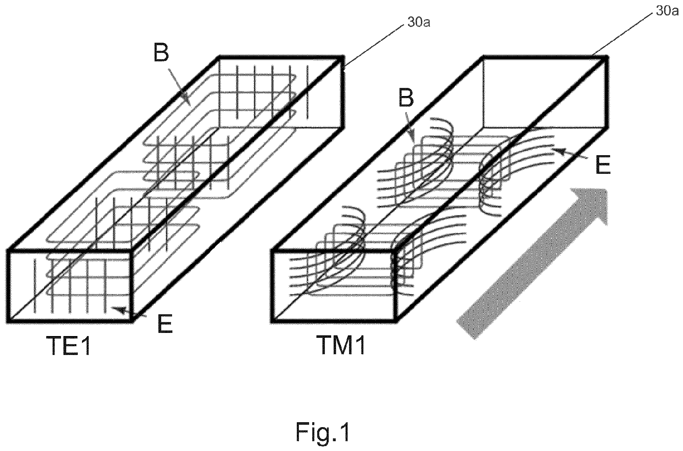

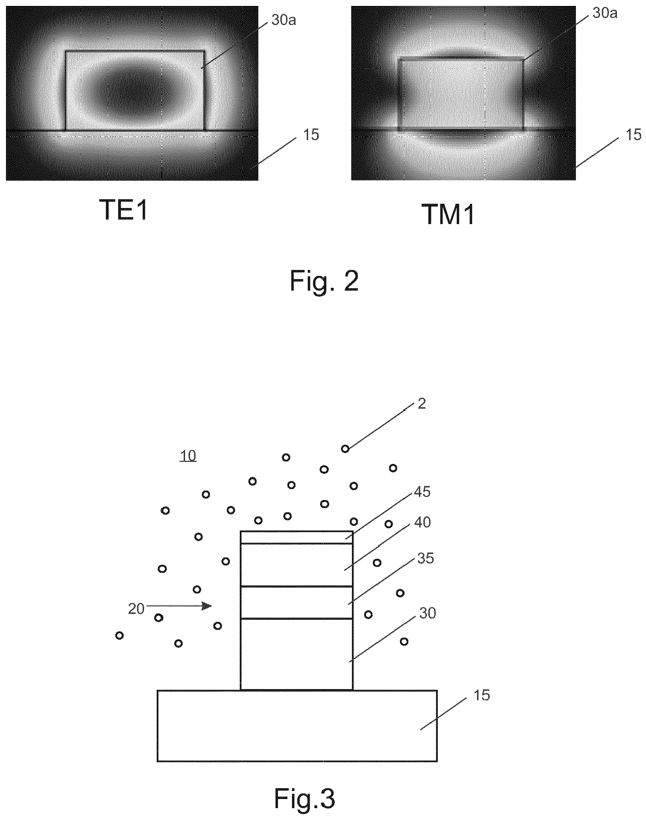

Next, some aspects relating to the optical sensor for detecting hydrogen are described in more detail. The optical sensor for detecting hydrogen is optically coupled to a light source for receiving light from the light source, which is preferably in the visible range or in the infrared range. The optical sensor generally comprises at least one Hybrid (photonic-) plasmonic waveguide (HPWG). HPWG are structures that use a combination of both the following two structures/principles:
1) A dielectric (or photonic) waveguide is a microstructure which is often used in photonics to guide light by total internal reflection, similar to an optical fiber. In contrast to optical fibers, however, dielectric waveguides do not generally have a circular cross-section, and are typically deposited, machined, or ion-implanted in/on a substrate. Known examples of such waveguides are Silicon-on-a-substrate (SOI) waveguides, that is, a Si waveguide, having a comparatively high refractive index, of rectangular cross-section is obtained by lithography on a SiO₂ substrate having a comparatively lower refractive index. The field lines for the magnetic field and the electric field for the TE1 and TM1 modes in an exemplary dielectric waveguide 30a are shown in Fig. 1, the arrow indicating the direction of light propagation of light coupled into the waveguide. These well-known field distributions of a dielectric waveguide are made use of, in a modified form, in the HPWG according to aspects, as is described further below. The electric field distributions for both modes are schematically shown in Fig. 2, with lighter colour indicating a higher electric field strength. In the TE1 mode, the electric filed is concentrated left and right to the waveguide (with the substrate being underneath the waveguide), wherein for the TM1 mode, the electric field is concentrated above and below the waveguide. In aspects of the invention, an additional (semi-)metallic sensing layer is positioned, typically together with a buffer layer, on top of the waveguide of Fig. 1 and Fig. 2, and thus in the area where a part of the electric field is concentrated for the TM1 mode. Hence, a change in the dielectric properties of the sensing layer - due to a change in the hydrogenation of the sensing layer material due to a varying hydrogen level/partial pressure in the surrounding medium - will influence the electric field of the TM1 mode, but hardly or not all the electric field of the TE1 mode. This is, amongst other effects described further below, employed in a sensor according to aspects.
2) Metallic (or plasmonic) waveguides are metallic microstructures on a dielectric. Light propagates near the metal surface, either at the metal-dielectric interface, or in a gap of the metal. A typical metal conventionally used is silver. Losses in metallic waveguides are typically much higher than in dielectric waveguides as described above, but the higher confinement allows for smaller structures like relatively sharp turns.

When combining the principles of 1) and 2) above, a hybrid (photonic-) plasmonic waveguide (HPWG) results, which is employed in embodiments. These are structures that use a combination of both structures 1) and 2) introduced above, resulting in some advantages. These are predominantly a better compromise between loss and confinement, and further, that different propagation modes are supported in different parts of the structure. The latter allows to use a HPWG as a polarizer for the light coupled into it.

As used herein, "substrate" is intended to mean a physical body having at least one, preferably substantially flat surface, on which the dielectric plasmonic-photonic waveguide (HPWG), and other elements stacked thereon, are provided according to embodiments. The substrate may typically comprise a dielectric such as SiO₂.

The optical sensor of embodiments has a sensing layer that changes its dielectric properties - predominantly, its absorption coefficient and/or its refractive index - with respect to the received light, depending on an amount of hydrogen present at the sensing layer, and thus with the hydrogenation level of the metal of the sensing layer. This means, a changing hydrogen partial pressure also leads to a change in dielectric properties of the sensing layer.

As stated above, the amount of hydrogen can qualitatively be defined in terms of a partial pressure of hydrogen at the optical sensor, which is directly related to an amount of hydrogen (in ppm) dissolved in the insulation liquid. Thereby, the absorption coefficient and/or refractive index of the optical sensor expresses/reflects the amount of hydrogen present at the sensing layer, and can therefore be taken as a status indicator of the electrical equipment - as a growing hydrogen concentration is an early indication for potential fault of the electrical equipment, as described further above.

According to aspects, a hydrogen sensor for detecting hydrogen in a medium to be monitored comprises a hybrid photonic-plasmonic waveguide (HPWG). The HPWG comprises a dielectric waveguide with a substantially rectangular cross-section, and a buffer layer stacked on top of the dielectric waveguide. A sensing layer is stacked on the buffer layer. It is metallic or semi-metallic and has a refractive index and/or absorption coefficient which is dependent on a hydrogen partial pressure at the sensing layer. An optional catalytic layer is stacked on the sensing layer. The hydrogen sensor is configured such that a medium to be monitored is in physical contact with the sensor, and further configured such that hydrogen in the medium may permeate through the catalytic layer into the sensing layer. At least one mode of a propagating wave in the HPWG is affected by a change in the refractive index and/or absorption coefficient of the sensing layer in dependence of, or due to, a change in the hydrogen concentration in the medium to be monitored.

According to aspects, the HPWG may be configured such that an incoming electromagnetic wave propagating in the HPGW has an electric field component of the TM1 mode in parallel to the sensing layer and an electric field component of the TE1 mode in a normal direction to the sensing layer. Predominantly, the TM1 mode is influenced by the effective refractive index and/or effective absorption coefficient of the sensing layer and catalytic layer stack. The TE1 mode, much less or practically not influenced, may be used as a reference signal which typically remains mainly constant. Typically, the sensing layer comprises a metal or semi-metal, which undergoes a chemical reaction in the presence of hydrogen, typically a hydrogenation, wherein a metal-hydride-complex is formed.

According to aspects, the sensing layer comprises at least one of Pd, WO₃, Mg-M2, or Mg-M1-M2, wherein M1 and M2 are transition metals or aluminium, more preferably at least one of Ti, Ni, Zr, and Ta. Typically, the dielectric waveguide comprises at least one of Si, GaAs, and AlGaAs. Typically, the buffer layer may comprise, as a non-limiting example, SiO₂. The catalytic layer may comprise at least one of Pd, Pt, and Ni, which may optionally be alloyed with at least one of gold, silver, and copper.

According to aspects, the dielectric waveguide may have a closed configuration, which is chosen from at least one of: a ring, an oval, a closed spiral, and a switched-back configuration. A sensor configuration according to the switched-back type is, e.g., described in D.-X. Xu, A.D., A. Delâge, P. Waldron, R. McKinnon, S. Janz, J. Lapointe, G Lopinski, T. Mischki, E. Post, P. Cheben and J.H. Schmid: "Folded cavity SOI microring sensors for high sensitivity and real time measurement of biomolecular binding", OPTICS EXPRESS, 2008. 16: p. 12. In other embodiments, the dielectric waveguide may be straight and have the shape of a slab.

According to aspects, the sensor may be configured such that a ratio between the amplitudes of the TM1 electric field component and the TE1 electric field component of a propagating wave in the HPWG are dependent on the hydrogen partial pressure at the sensing layer. The ratio can hence be employed as a measure for the hydrogen concentration in the medium to be monitored. Further, a resonance frequency in the HPWG having a ring-shape may be dependent on the refractive index of the sensing layer, and hence the resonance frequency may be taken as a measure for the hydrogen concentration in the medium to be monitored. The hydrogen concentration is proportional to the hydrogen partial pressure, which may thus be easily deduced from each other, as is known to the skilled person.

In aspects, the dielectric waveguide may generally have a width, in parallel to the plane of the sensing layer, from 100 nm to 4 µm, more preferably from 300 nm to 2 µm. The ratio between that width of the dielectric waveguide and the typically applied electromagnetic wavelength may be from about 0,24 to about 0,40.

In aspects, the HPWG may have a closed configuration, such as the shape of a ring, and a coupling waveguide is provided adjacent to a side face of the HPWG. A wave propagating in the coupling waveguide is at least partially coupled into the HPWG. The TM1 mode, or the TE1 mode, depending on the sensor configuration, of a propagating wave in the HPWG is then influenced primarily by the refractive index of the sensing layer of the HPWG. This leads to a dependency of the resonance frequency on the hydrogen partial pressure at the sensing layer, which may hence be used as an indicator for a hydrogen concentration in the medium. Light from a light source is coupled into the coupling waveguide, and is partially coupled into the HPWG through its sideface. The amount of light which is coupled into the HPWG is dependent on the condition if the resonance frequency of the HPWG ring is met by the coupled light - or on the deviation of the frequency of the light from the light source versus the resonance frequency of the ring. Thus, when the resonance frequency of the HPWG ring changes due to a changing hydrogen level in the surrounding medium, leading to a changing refractive index of the sensing layer of the HPWG ring, this can be measured via a changing intensity of the light which passes the coupling waveguide without being coupled into the HPWG. Thus, in aspects, a photodetector (or spectrometer) is positioned at an end of the coupling waveguide which is opposite to the light source. A control unit operatively coupled to the photodetector may then determine the hydrogen level in a surrounding medium based on, e.g., a look-up table with pre-stored photodetector readings versus the respective hydrogen partial pressure in the medium. In the previous case, the light source may, in a non-limiting example, be a laser diode. Also, the light source may be a tunable laser, which is controlled by a control unit to continuously sweep a (narrow) frequency range around the resonance frequency of the HPWG, and wherein the photodetector detects the light intensity of the light which is not coupled into the HPWG.

In aspects, the HPWG may have the shape of a slab. The electromagnetic wave may be coupled into the dielectric waveguide of the slab at one of its ends, and the TM1 mode of the propagating wave is in this case influenced primarily by the absorption coefficient of the sensing layer. An attenuation of the TM1 mode in the slab may thus be used as an indicator for a hydrogen concentration in the medium. In a sensing system according to aspects, employing such a sensor, light from a light source is coupled into the slab. The light may preferably be unpolarised, or may also have a defined known polarization. While passing the sensor in form of the slab, the slab acts as a polarization selector. While the TE1 mode is transmitted substantially without attenuation, or with only a small attenuation which may be determined during a calibration process, the TM1 mode is attenuated, as described, in dependency of the absorption coefficient of the sensing layer, and thus in dependency of the hydrogen partial pressure in a surrounding medium. A light detection device at the end of the slab opposite of the light source is configured to detect the intensity of the TM1 mode and of the TE1 mode after transmission, meaning the light detection device is suitable to detect intensities of light with different polarization. In a non-limiting example, the light detection device may comprise a polarizing beam splitter, typically at least one of: a polarizing beam splitter cube, a Wollaston prism, a Glan-Thomson prism, a Glan-Laser prism, and any combination of the former. Typically, two photodetectors are provided for the two polarization components exiting the polarizing beam splitter, representing the TM1 mode and TE1 mode, respectively. The skilled person will readily understand that there exists a large number of light detection devices which are suitable for the above sensing system, and which are regarded to be included in the scope of the present disclosure. The sensing system further comprises a control unit operatively coupled to the photodetectors. The ratio between the signals of the photodetectors is indicative of the hydrogen partial pressure (or concentration) in the medium surrounding the sensor (slab). The control unit is configured to determine/calculate the hydrogen partial pressure in the medium, based on stored values of the ratio of the intensities of the two polarization components of the TE1 mode and the TM1 mode, as measured by the two photodetectors after the polarizing beam splitter.

In aspects, the dielectric waveguide may be provided on a dielectric substrate. A buffer layer, and optionally the sensing layer, may be provided to extend over the side borders of the dielectric waveguide in a lateral direction. In such configurations, the dielectric waveguide is embedded between the dielectric substrate and the buffer layer.

According to aspects, a hydrogen sensing system comprises a sensor according to any of the above aspects, a light source for coupling light into the sensor, and at least one light detection device. The hydrogen sensing system may be used in an electrical installation such as a power transformer.

According to an aspect, an optical gas/hydrogen sensing system, in particular a control unit comprised therein operatively coupled to a light detection device, may further comprise a network interface for connecting the system to a data network, in particular a global data network. The data network may be a TCP/IP network such as the Internet. The system is operatively connected to the network interface for carrying out commands received from the data network. The commands may include a control command for controlling the system to carry out a task such as a measurement, a self-test, a calibration, or the like. In this case, the control unit is adapted for carrying out the task in response to the control command. The commands may include a status request. In this case, the control unit may be adapted for sending measurement data and/or status information to the network interface, and the network interface is adapted for sending the information over the network, e.g., in response to a status request. The commands may include an update command including update data. In this case, the control unit is adapted for initiating an update in response to the update command and using the update data.

According to aspects, an electrical equipment with an insulation liquid is provided, wherein a hydrogen sensor of the optical hydrogen sensing system as described above is immersed in the insulation liquid, i.e. partially immersed, so that the optical sensor is at least in partial contact with the insulation liquid.

### Detailed Description of the Figures and Exemplary Embodiments

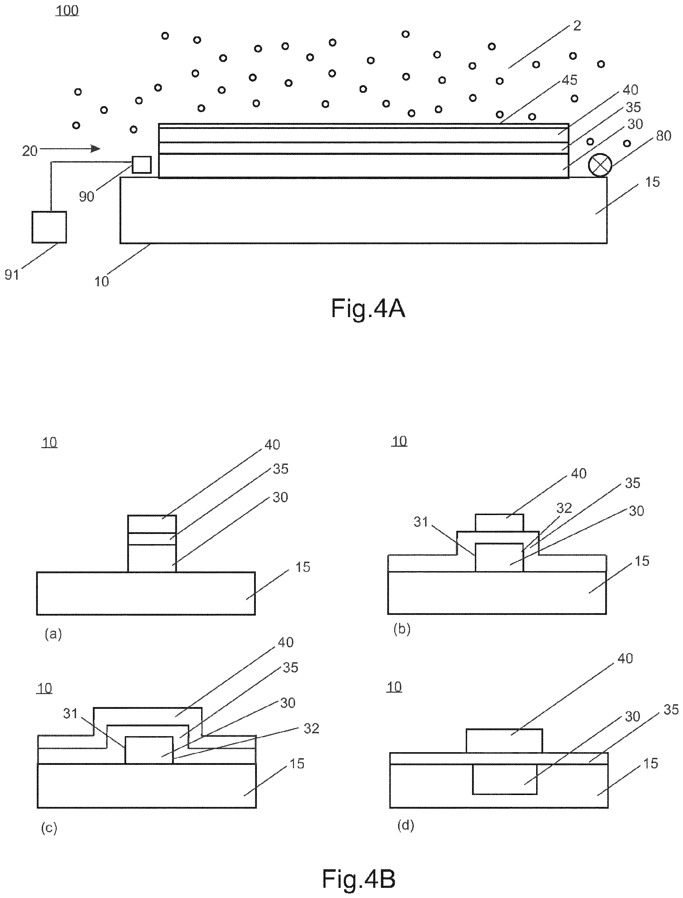

Fig. 3 shows an exemplary hydrogen sensor 10 for detecting hydrogen in a medium 2 to be monitored according to embodiments. The sensor comprises a hybrid photonic-plasmonic waveguide (HPWG) 20. It comprises a dielectric waveguide 30 with a substantially rectangular cross-section, typically provided on a dielectric substrate 15. A buffer layer 35 is typically stacked on top of the dielectric waveguide 30. A sensing layer 40 is stacked on the buffer layer 35, wherein the sensing layer 40 may be metallic or semi-metallic. Optionally, a catalytic layer 45 may be stacked on the sensing layer 40, or may in embodiments be integrated with the sensing layer 40. In Fig. 4A, a side view on the hydrogen sensor 10 of Fig. 3 is shown, wherein the sensor 10 is part of a hydrogen sensing system 100. Additionally to the sensor 10 of Fig. 3, light from a light source 80 is coupled into one end of the dielectric waveguide 30 during operation and measured, after passing it, with a light detection device 90. In Fig. 3 and Fig. 4A and 4B, the HPWG 20 has the elongated shape of a slab.

In a hydrogen sensing system 100 according to embodiments shown in Fig. 4A, employing for example the sensor 10 of Fig. 3, a light detection device 90 at the end of the slab opposite of the light source 80 is configured to detect the intensity of the TM1 mode and of the TE1 mode after transmission, meaning the light detection device 90 is suitable to detect intensities of light with different polarization. In a non-limiting example, the light detection device 90 may comprise a polarizing beam splitter (not shown), typically at least one of: a polarizing beam splitter cube, a Wollaston prism, a Glan-Thomson prism, a Glan-Laser prism, and any combination of the former. Typically, the light detection device 90 comprises two photodetectors (not shown) provided for the two polarization components exiting the polarizing beam splitter, and representing the TM1 mode and TE1 mode, respectively. The skilled person will readily understand that there exists a large number of light detection devices which are suitable for such a hydrogen sensing system, and which are regarded to be included in the scope of the present disclosure. The sensing system further comprises a control unit 91 operatively coupled to the photodetectors. The ratio between the signals of the photodetectors is indicative of the hydrogen partial pressure (or concentration) in the medium 2 surrounding the sensor (slab) 10. The control unit 91 is configured to determine/calculate the hydrogen partial pressure in the medium 2, based, e.g., on stored values of the ratio of the intensities of the two polarization components of the TE1 mode and the TM1 mode, as measured by the two photodetectors after the polarizing beam splitter of the light detection device 90.

Fig. 4B shows examples of sensors according to embodiments, similar as shown in Fig. 3, in a cross-sectional view (medium 2 not depicted for illustrational purposes). Variant (a) shows a (typically: Silicon-on-insulator) sensor 10 with a waveguide 30 with a top buffer layer 35 and a sensor layer 40, stacked. Variant (b) shows a (typically: Silicon-on-insulator) sensor 10 with a waveguide 30 with a fully covering buffer layer 35 and a top sensor layer 40. Variant (c) shows a (typically: Silicon-on-insulator) sensor 10 with a waveguide 30 with covering buffer layer 35 and a covering sensor layer 40. Variant (d) shows a sensor 10 with a waveguide 30 such as may typically be produced by ion implantation, with covering buffer layer 35 and a sensor layer 40 of variable width, which may typically be larger than the width of the waveguide. As can be seen in Fig. 4B variant b) and c), the buffer layer 35 and optionally the sensing layer 40 may be provided to extend over the side borders 31, 32 of the dielectric waveguide 30 in a lateral direction. This may result in the dielectric waveguide 30 being embedded between the dielectric substrate 15 and the buffer layer 35. In all variants of Fig. 4B, an additional catalytic layer 45 and a coating layer may typically be provided similar to Fig. 3.

In these embodiments, which may be described to be based on polarization selection, predominantly changes of the absorption coefficient of the sensing layer 40 due to a changing hydrogen level in the medium 2 are determined. The buffer layer 35 and the substrate 15 typically have a lower refractive index than the dielectric waveguide 30 to ensure proper guiding of the light coupled into the HPWG 20. Typically, the sensor stack, comprising the sensing layer 40 and optionally an extra dedicated catalytic layer 45, may have a higher refractive index (at the wavelength of the light used/coupled in) than the buffer layer 35. All layers typically have a higher refractive index than the environment/surroundings. The effective absorption coefficient of the sensor stack varies due to an external parameter present in the environment of the sensor, which is typically the hydrogen partial pressure or the partial pressure of a different gas in a surrounding medium 2.

Next, some observations of the working principle of a sensor 10 according to Fig. 3 and Fig. 4A/4B are provided. Generally, the TE1 mode is guided by the dielectric waveguide 30, and its intensity is relatively insensitive to the sensing layer 40 or sensing stack. Thus, the TE1 mode may typically be employed as a reference for the light intensity.

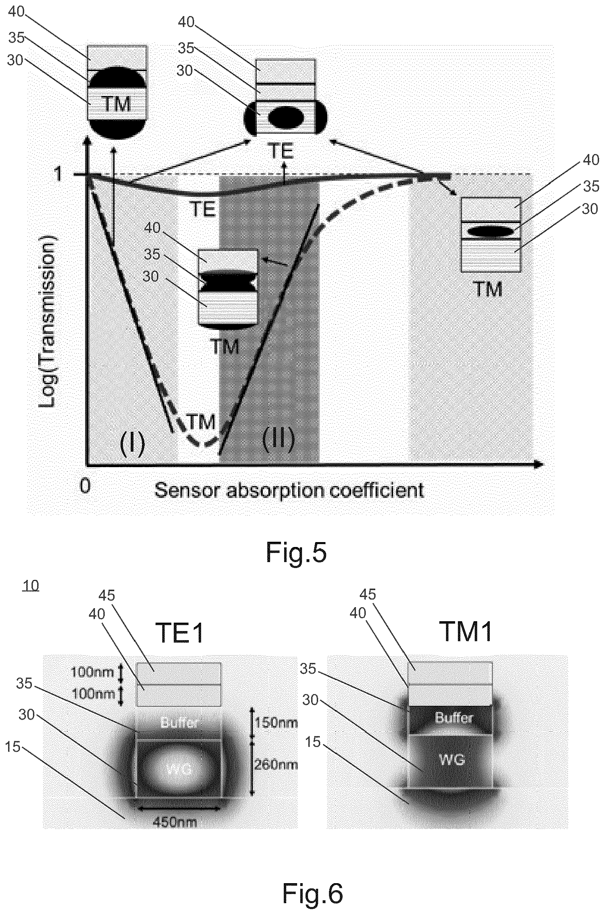

Contrary to the above, the TM1 mode is mostly guided within the buffer layer 35 (buffer region), as was previously described. It is therefore in first order sensitive to a variation of the absorption coefficient of the sensing layer 40 or sensor stack. The attenuation per length of the HPWM 20 may be tuned by changing the thickness of the buffer layer 35 - whereby the shape of the function "transmission of the TM1 mode as a function of sensor absorption coefficient" remains unchanged. The TM1 mode is also in second order sensitive to the refractive index of the sensor stack. As is shown in Fig. 5, the attenuation per length of HPWG 20 is always significantly lower for the transverse electric (TE1) propagation mode - and thus the transmission is higher - than for the transverse magnetic propagation mode, and this for all values of the absorption coefficient of the sensing layer/sensor stack. Hence, the transmission of the TE1 mode may be used in embodiments as an internal light intensity reference, and the transmission of the TM1 mode as the sensing signal.

Fig. 5 further shows the principle of operation and sensing ranges of a HPWG sensor 10 according to embodiments. The transmitted light intensity is shown as a function of the optical absorption coefficient of the sensor stack (for constant refractive index), whereby the catalytic layer 45 is not shown. In Fig. 5, TE means transverse electric mode, TM means transverse magnetic mode.

Thereby, two basic modes of operation can be discriminated, the sensing ranges being indicated in Fig. 5:

### Sensing range (I): dielectric waveguide operation

For a sensor stack with low absorption coefficient, both TE1 mode and TM1 mode are guided by the dielectric waveguide 30. The evanescent wave of the TM1 mode is situated above and below the waveguide 30, in the buffer layer 35, respectively in the substrate 15 (not shown in Fig. 5). For a well-dimensioned buffer layer 35, a small part of the upper evanescent wave is within the sensing layer 40 / sensor stack, too. Therefore, a small increase of the absorption coefficient of the sensor stack absorbs the top part of the upper evanescent wave, resulting in a strong attenuation of the TM1 mode transmitted intensity. The TE1 mode is marginally affected, as it is situated within the dielectric waveguide 30. Its evanescent wave is left, respectively right of the dielectric waveguide, where the sensor stack according to embodiments is typically absent / not present.

### Sensing range (II): hybrid waveguide operation

For increasing values of the absorption coefficient of the sensing layer 40 / sensor stack according to embodiments, two mechanisms, namely (i) absorption losses in the sensor stack, and (ii) increased confinement within the buffer layer 35 are counteracting each other. For sufficient high absorption coefficient of the sensor stack, the transmission of the TM1 mode increases with increasing absorption coefficient due to the better confinement within the buffer layer 35: this is the hybrid waveguide operation, because transmission of the TE1 mode is controlled by the dielectric waveguide 30, while the TM1 transmission is controlled by the increasing metallicity of the sensor stack, thus acting partially as a metallic waveguide.

In a hybrid photonic-plasmonic waveguide HPWG 20 according to embodiments, which is employed for sensing hydrogen by variation of optical absorption coefficient (Polarization selector), the effective absorption coefficient of the sensing layer 40 / sensor stack varies with the hydrogen concentration - from a strong metallic character (no hydrogen content in the surroundings and in the sensing layer, high optical absorption) to a strong insulator character (fully hydrogenated sensing layer, low optical absorption).

The sensor stack comprises a sensing layer 40, and typically, but not necessarily also a coating stack stacked on the sensing layer 40. The coating stack comprises the catalytic layer 45 deposited on the sensing layer 40 for catalytic splitting of the hydrogen molecules, and for fostering permeation of so-produced atomic hydrogen towards the sensing layer 40. Typical catalytic layers 45 comprise, as non-limiting examples, palladium, platinum or nickel, optionally further alloyed with gold, silver, and/or copper, to name a few options. The catalytic layer may be coated / overlayed further by other layers, such as PTFE. Their function may be to block diffusion of poisoning gases (e.g. CO, CH₄, H₂S) and to reduce the sensitivity to side reactions like a water formation from the hydrogen atoms in the presence of oxygen traces in the sensing layer 40 and catalytic layer 45.

Fig. 6 shows an exemplary sensor 10 with field distributions of the electric field for the TE1 mode and the TM1 mode. Different to other figures herein, a darker colour is representative to a stronger field strength. Non-limiting, exemplary parameters are SiO₂ with n = 1.5 as a substrate 15. The dielectric waveguide 30 comprises Si with n = 3.44. The sensor stack comprises a sensing layer 40 comprising Mg₇₀Ti₃₀Hₓ, or alternatively Mg₅₂Ni_{20.5}Zr_{27.5}Hₓ. The coating stack comprises a catalytic layer 45 with Pd having n = 3.2 and k = 8.15. Around the sensor 10, an insulation oil of a transformer may be provided as the medium 2 to be monitored.

For the sensing range I as described above, the dielectric waveguide operation, a non-limiting example for a hydrogen sensitive material of the sensing layer 40 is proton-intercalated tungsten trioxide. For the sensing range II as described above, the hybrid waveguide operation, examples of a sensing layer 40 that is sensitive to hydrogen are Mg-Ni-Zr or Mg-Ti, both coated/stacked with a catalyst layer 45 comprising Pd.

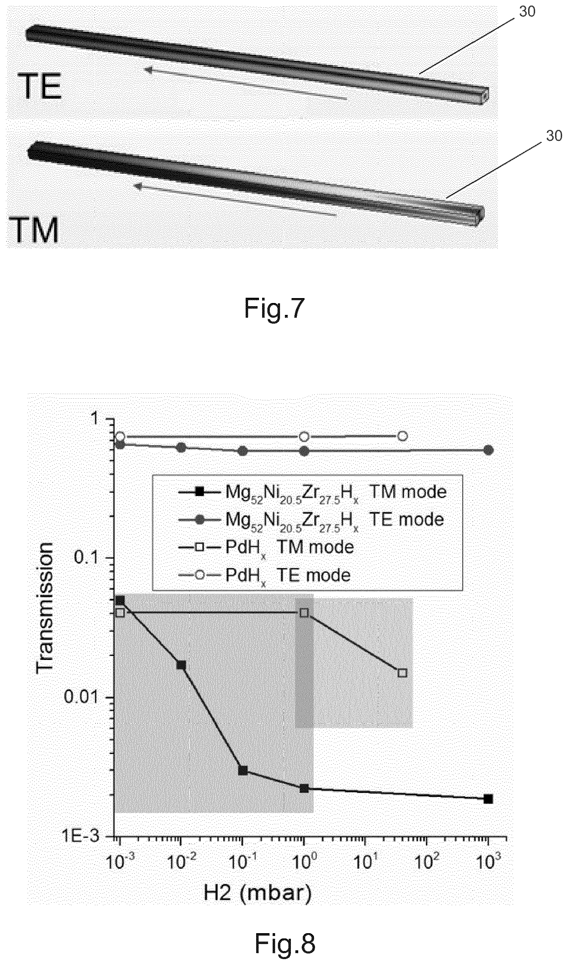

Fig. 7 shows the waveguide 30 of a sensor 10 according to embodiments, such as shown in Fig. 3 and Fig. 4A/4B and as described above. Depicted are simulations for the decrease of the TE1 and the TM1 mode when light is coupled in the sensor 10, having the shape of a slab, from the right side.

Some of the advantages of a sensing system employing a sensor 10 according to embodiments as described above are the possibility to add as many protection layers on top of the sensor stack to ensure a robust sensor that is only sensitive to the desired species and resistant to adverse species and corrosion. The system is cost effective, as it is suitable to work at, in a non-limiting example, a telecom wavelength of 1550 nm, for which generally, low-cost components are available. The sensitivity is generally higher than with a Pd-based sensor, and it has a high suitability to detect hydrogen in oil of a transformer.

In Fig. 8, detection ranges and sensitivities for optical transmission and hydrogen pressure operation range are shown at room temperature (25°C) compared for Mg₅₂Ni_{20.5}Zr_{27.5}Hx as a sensing layer 40 such as in embodiments, and for PdHₓ as a sensing layer 40. As can be seen, the transmission of the TE1 mode is hardly affected with hydrogen partial pressure for the Mg-based sensor, while the transmission of the TM1 mode is greatly varying with hydrogen partial pressure for the Mg-based sensor. Thereby, the Mg-based sensor has generally a much better sensitivity at lower hydrogen partial pressures (left grey shaded area), whereas the Pd-based sensor shows hardly any sensitivity in this range and is only responsive at higher hydrogen partial pressures (upper right grey shaded area).

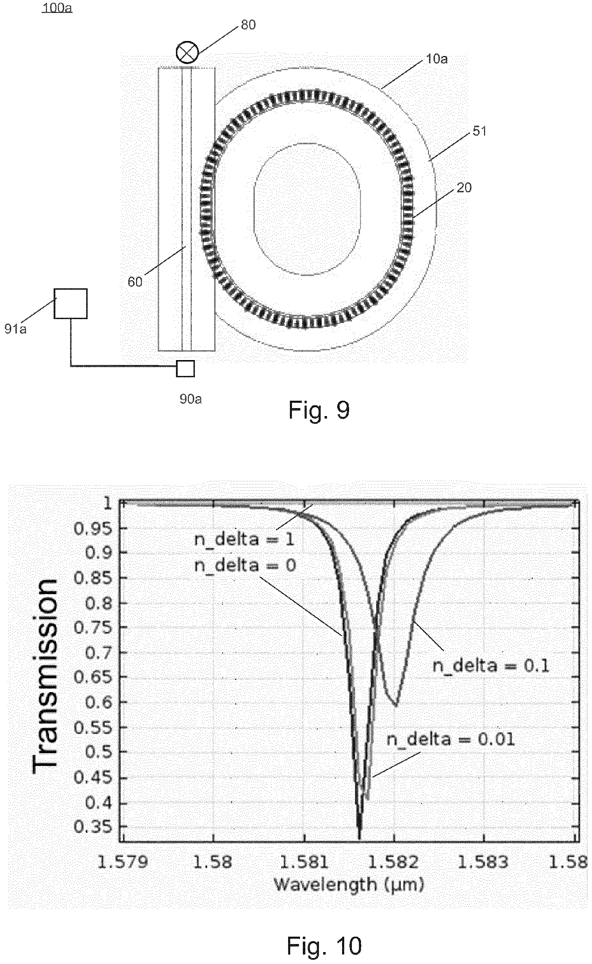

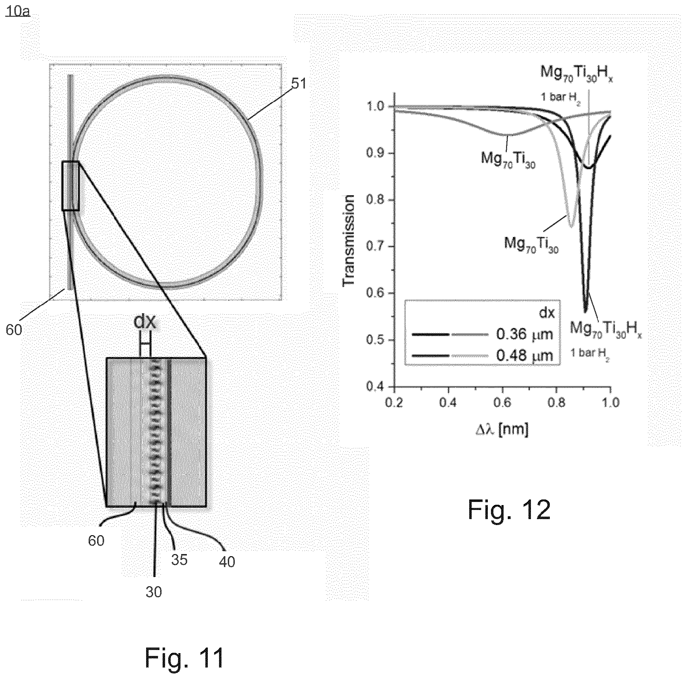

In Fig. 9, an embodiment of a sensor 10a is shown, having a different shape and light path than in the previous embodiments. The sensor 10a is also shown as part of a sensing system 100a, which further comprises a light source 80, a light detection device 90a, and a control unit 91a. The HPWG 20 and thereby also the waveguide 30 have a closed configuration. That is, light in the HPWG 20 is travelling around in a ring 51, an oval, a closed spiral, or similar. In order to couple the light into the HPWG 20, a coupling waveguide 60 is provided on a side face of the HPWG 20. In Fig. 9, the ring 51 may exemplarily and non-limiting have a diameter of about 5 µm. The alternating minima and maxima of the field along the ring 51 (not to scale) indicate the wave inside the sensor 10a. Further details on the structure of the waveguide 20 are provided with respect to Fig. 11. Generally, such closed- or ring configurations may have effective, meaning average diameters from about 2 µm to 50 µm, more particularly 3 µm to 15 µm. It is understood that these are no hard limits, as they are also dependent on the wavelength employed.

This sensor 10a is typically employed for sensing variations of the refractive index as a ring resonator. With this closed configuration of, e.g., a ring 51 architecture, a frequency-dependent resonance of the transmitted light within the ring waveguide structure is obtained.

In operation according to embodiments, a light wave propagating in the coupling waveguide 60, coming from the light source 80 being e.g. a LED, is at least partially coupled into the HPWG 20 of the sensor 10a. The TM1 mode, or the TE1 mode, depending on the sensor configuration, of a propagating wave in the HPWG is then influenced primarily by the refractive index of the sensing layer 40 of the HPWG. This leads to a dependency of the resonance frequency on the hydrogen partial pressure at the sensing layer, which may hence be used as an indicator for a hydrogen concentration in a surrounding medium. Light from the light source 80 is coupled into the coupling waveguide 60 and is partially coupled into the HPWG 20 through its sideface. The amount of light which is coupled into the HPWG is, inter alia, dependent on the condition, if the resonance frequency of the HPWG ring is met by the coupled light - or on the deviation of the (middle or main) frequency of the light from the light source versus the resonance frequency of the ring 51. Thus, when the resonance frequency of the HPWG ring changes due to a changing hydrogen level in the surrounding medium, leading to a changing refractive index of the sensing layer 40 of the ring 51, this can be measured via a changing intensity of the light which passes the coupling waveguide 60 without being coupled into the HPWG 20. Thus, in embodiments, a light detection device 90a, such as exemplarily a photodetector or spectrometer (both not shown in detail), is positioned at an end of the coupling waveguide 60 which is opposite to the light source 80 which emits preferably unpolarised light. A control unit 91a operatively coupled to the photodetector may then determine the hydrogen level in a surrounding medium based on, e.g., a look-up table with pre-stored photodetector readings versus the respective hydrogen partial pressure in the medium. In the previous embodiments, the light source may, in a non-limiting example, be a laser diode. Also, the light source may be a tunable laser, which is controlled by a control unit to continuously sweep a (narrow) frequency range around a resonance frequency (without hydrogen being present, or at a defined hydrogen partial pressure) of the HPWG 20 of the sensor 10a, and wherein the photodetector detects the light intensity of the light which is not coupled into the HPWG, by employing the readings from the light detection device 90. From the detected shift of the resonance frequency with changing hydrogen partial pressure, the control unit 91a may derive the current hydrogen partial pressure in the surrounding medium 2 using, e.g., a look-up-table.

In Fig. 10, changes of the resonance frequency are depicted for various changes in the refractive index of the sensing layer 40 of sensor 10a. A more detailed view of a sensor 10a with closed configuration is shown in Fig. 11. In the detailed enlarged section below, it can be seen that the coupling waveguide 60 is distanced by a small distance dx from the dielectric waveguide 30 of the HPWG 20. In Fig. 12, the dependency of the shift of the transmission in the ring 51 from the change of the resonance wavelength is depicted. This configuration is mainly sensitive on the TE1 mode. For sensing hydrogen with the required finesse of the resonance (below 1 nm at full-width at half maximum FWHM) for a 50-micrometer-radius race-track absorption sensor such as in Fig. 11, Mg₇₀Ti₃₀ may exemplarily be employed as material of the sensing layer 40. The shift of the resonance frequency can be tuned by the distance dx between the straight part of the coupling waveguide 60 and the ring 51 part of the resonator. If the sensing layer 40 is, in embodiments, inside of the loop formed by the ring 51 resonator as in Fig. 7 and 8, it is sensitive to the TE-like propagation mode. Further embodiments include that the sensing layer 40 is on the outside of the dielectric waveguide 30 respectively the HPWG 20. In that case, the sensing layer 40 is predominantly sensitive to the TM1 mode.

All sensors 10, 10a and sensing/detection systems 100, 100a according to embodiments described with respect to the figures may exhibit the properties and parameters as described in the aspects section further above, unless technically not feasible. It goes without saying that the embodiments described with respect to the figures may be greatly varied and modified, with some possible parameter ranges and constructional options being provided in the aspect section, and that such modifications are regarded to fall under the scope of this disclosure.

It is understood that the described sensors 10, 10a and detection systems 100, 100a have to be characterized prior to their use, or at least in prototype status, in order to obtain the data mentioned above about the relation between hydrogen partial pressure in a surrounding medium, and response of the optical sensor. As such optical sensors can be reproduced with high precision, this might only be necessary for a prototype. The obtained data then may be reused in the mass production process.

Although specific features of various embodiments of the invention may be shown in some drawings and not in others, this is for convenience only. In accordance with the principles of the invention, any feature of a drawing may be referenced and/or claimed in combination with any feature of any other drawing.

This written description uses examples to disclose the invention, including the best mode, and also to enable any person skilled in the art to practice the invention, including making and using any devices or systems and performing any incorporated methods. While various specific embodiments have been disclosed in the foregoing, those skilled in the art will recognize that the spirit and scope of the claims allows for equally effective modifications. Especially, mutually non-exclusive features of the embodiments described above may be combined with each other. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A hydrogen sensor (10, 10a) for detecting hydrogen in a medium (2) to be monitored, the sensor comprising:
- a hybrid photonic-plasmonic waveguide (HPWG) (20), comprising:
o a dielectric waveguide (30), having a substantially rectangular cross-section;
o a buffer layer (35), stacked on top of the dielectric waveguide (30),
o a sensing layer (40) stacked on the buffer layer (35), being metallic or semi-metallic and having a refractive index and/or absorption coefficient which is dependent on a hydrogen partial pressure at the sensing layer (40),
∘ optionally a catalytic layer (45) stacked on the sensing layer (40),
wherein the hydrogen sensor (10, 10a) is configured such that a medium (2) to be monitored is in physical contact to the sensor (10, 10a), and configured such that hydrogen in the medium (2) may permeate into the sensing layer (40), and wherein at least one mode of a propagating wave in the HPWG (20) is affected by a change in the refractive index and/or absorption coefficient of the sensing layer (40) due to a change in the hydrogen concentration in the medium (2) to be monitored.

2. The hydrogen sensor (10, 10a) of claim 1, wherein the HPWG (20) is configured such that an incoming electromagnetic wave propagating in the HPGW has an electric field component of the TM1 mode in parallel to the sensing layer (40), and has an electric field component of the TE1 mode in a normal direction to the sensing layer (40), and wherein predominantly the TM1 mode is influenced by the effective refractive index and/or effective absorption coefficient of the layer stack comprising a sensing layer (40) and optionally a catalytic layer (45), while the TE1 mode may be used as a reference signal.

3. The hydrogen sensor (10, 10a) of claim 1 or 2, wherein the sensing layer (40) comprises a metal or semi-metal which undergoes a chemical reaction in the presence of hydrogen.

4. The hydrogen sensor (10, 10a) of claims 1 to 3, wherein
- the sensing layer (40) comprises at least one of Pd, WO₃, Mg-M2, or Mg-M1-M2 where M1, M2 are transition metals or aluminium, preferably at least one of Ti, Ni, Zr, and Ta;
- wherein the dielectric waveguide (30) comprises Si, or GaAs, or AlGaAs; and
- wherein the buffer layer (35) comprises SiO₂; and
- wherein the catalytic layer (45) comprises at least one of: Pd, Pt, and Ni, which are optionally alloyed with at least one of: gold, silver, and copper.

5. The hydrogen sensor (10, 10a) of any preceding claim, wherein the dielectric waveguide (30) has:
a. a closed configuration, chosen from at least one of: a ring (51), an oval, a closed spiral, and a switched-back configuration, or
b. the shape of a slab.

6. The hydrogen sensor (10, 10a) of any preceding claim, wherein either:
a. a ratio between the amplitudes of the TM1 electric field component and the TE1 electric field component of a propagating wave in the HPWG (20) is dependent on the hydrogen partial pressure at the sensing layer (40) and can be employed as a measure for the hydrogen concentration in the medium (2) to be monitored; or
b. a resonance frequency in the HPWG (20) having a closed configuration is dependent on the effective refractive index of the layer stack comprising the sensing layer (40) and the catalytic layer (45) .

7. The hydrogen sensor (10, 10a) of any preceding claim, wherein the dielectric waveguide (30) has a width, in parallel to the sensing layer (40), from 100 nm to 4 µm, more preferably from 300 nm to 2 µm.

8. The hydrogen sensor (10, 10a) of any preceding claim, wherein the ratio between the width of the dielectric waveguide (30) and the typically applied electromagnetic wavelength is about 0,24 to about 0,40.

9. The hydrogen sensor (10a) of any preceding claim, wherein the HPWG (20) has a closed configuration, and wherein a coupling waveguide (60) is provided adjacent to a side face of the HPWG (20), so that a wave propagating in the coupling waveguide (60) is at least partially coupled into the HPWG (20), and wherein the TM1 or the TE1 mode of a propagating wave in the HPWG is influenced primarily by the refractive index of the sensing layer (40) of the HPWG, leading to a dependency of the resonance frequency on the hydrogen partial pressure at the sensing layer (40), which may be employed as an indicator for a hydrogen concentration in the medium (2).

10. The hydrogen sensor (10) of any preceding claim, wherein the hybrid HPWG (20) has the shape of a slab, and wherein an electromagnetic wave may be coupled into the slab at one end, and wherein the TM1 mode of the propagating wave is influenced primarily by the absorption coefficients of the sensing layer (40) and catalytic layer (45), so that an attenuation of the TM1 mode in the slab may be used as an indicator for a hydrogen concentration in the medium (2).

11. The hydrogen sensor (10, 10a) of any preceding claim, wherein the dielectric waveguide (30) is provided on a dielectric substrate (15).

12. The hydrogen sensor (10) of claim 11, wherein the buffer layer (35) and optionally the sensing layer (40) may be provided to extend over the side borders (31, 32) of the dielectric waveguide (30) in a lateral direction, and wherein the dielectric waveguide (30) is embedded between the dielectric substrate (15) and the buffer layer (35).

13. A hydrogen sensing system (100, 100a), comprising:
a. a sensor (10, 10a) according to any of claims 1 to 12,
b. a light source (80),
c. a light detection device (90, 90a);
further comprising a control unit (91, 91a) operatively coupled to the light detection device (90, 90a), and preferably a network interface for connecting the control unit (91, 91a) to a data network, wherein the control unit (91, 91a) is operatively connected to the network interface for carrying out commands received from the data network, and/or to send measurement data to the data network.

14. A method of detecting hydrogen in a medium (2) to be monitored, the method comprising:
- Providing a hydrogen sensor (10, 10a) according to any of claim 1 to 12, comprising a hybrid photonic-plasmonic waveguide (HPWG) (20) with a sensing layer (40), wherein the hydrogen sensor (10) is configured such that a medium (2) to be monitored is in physical contact with the sensor (10, 10a), and
- coupling an electromagnetic wave into the HPWG (20),
wherein the hydrogen sensor (10, 10a) is configured such that a medium (2) to be monitored is in physical contact to the sensor, wherein hydrogen permeates into the sensing layer (40) and influences at least one mode of a propagating wave in the HPWG (20) via the refractive index and/or absorption coefficient of the sensing layer (40).

15. The method of claim 13, wherein the HPWG (20) is configured such that an incoming electromagnetic wave propagating in the HPGW has an electric field component of the TM1 mode in parallel to the sensing layer (40), and has an electric field component of the TE1 mode in a normal direction to the sensing layer (40), and wherein the TM1 mode is influenced by the refractive index and/or absorption coefficient of the sensing layer, such that a measured relation between the TM1 component and the TE1 component of a propagating wave is dependent on the hydrogen partial pressure and is employed as a measure for the hydrogen concentration in the medium (2) to be monitored.
